# EUROPEAN PATENT SPECIFICATION

(11) **EP 0 275 202 B1**
(45) Date of publication and mention of the grant of the patent: **28.04.1993**
(21) Application number: 88300294.1
(22) Date of filing: 14.01.1988
(51) Int. Cl.: C12N 15/53, C12N 9/02, A61K 37/50, A61K 7/00, C12N 1/18

(54) **Thermostable human Cu/Zn superoxide dismutase muteins**
Thermostabile menschliche Cu/Zn-Superoxid-Dismutase-Muteine
Mutéines thermostables de superoxyde dismutase Cu/Zn humaine

(30) Priority: 15.01.1987 US 3578
(43) Date of publication of application: 20.07.1988
(73) Proprietor: CHIRON CORPORATION, Emeryville, California 94608 (US)
(72) Inventor: Hallewell, Robert A., San Francisco California 94109 (US); Tekamp-Olson, Patricia, San Francisco California 94122 (US)
(74) Representative: Goldin, Douglas Michael

(56) References cited:
- EP-A- 0 218 480
- DE-A- 3 628 508
- BIOCHEMISTRY, vol. 19, 1980, pages 2310-2316, American Chemical Society, Easton, US; J.R. JABUSCH et al.: "Some sulfhydryl properties and primary structure of human erythrocyte superoxide dismutase"

## Description

### Technical Field

This invention is in the fields of protein chemistry and genetic engineering. More particularly it relates to muteins of human Cu/Zn superoxide dismutase in which either or both of the free cysteines at amino acid positions 6 and 111 of the native protein have been replaced with a neutral amino acid.

### Background

Superoxide dismutases are a family of enzymatic proteins that include associated metal ions. The members of the family differ in both amino acid sequence and in the type of associated metals. Their ability to catalyze the destruction of superoxide ions renders them useful as pharmaceuticals, in cosmetics, and in food preservation. The amino acid sequence of human Cu/Zn superoxide dismutase (hereinafter referred to as hSOD) is described in Jabusch et al, Biochemistry (1980) 19:2310-2316. The cloning and sequencing of hSOD cDNA and the production of hSOD in bacteria and yeast are described in EPA 84111416.8 (published 24 April 1985 under number 0 138 111).

One potential shortcoming of hSOD is its relative lability to heat. This property limits its shelf life and its ability to be made into pharmaceutical, food, or cosmetic formulations that may be subjected to elevated temperatures. In this regard, it is known that yeast Cu/Zn SOD contains no free cysteines, but is less thermostable than bovine SOD or hSOD (Steinman, H. M. in Superoxide Dismutase Vol I, pp 18-19, CRC Press, 1982). The only reference relating to increasing the thermal stability of hSOD is Jabusch et al, supra. It reports that alkylation of Cys 111 of native hSOD with iodoacetate yields a more stable molecule.

The only reference that describes a mutein of hSOD is Hallewell et al, Nucleic Acids Research (1985) Vol 13, No 6, pp 2017-2034 . It reports a hSOD mutein having an Ala → Glu substitution at position four. No dismutation activity was detected for that mutein and it was thus estimated that its activity was reduced more than 20-fold relative to the native molecule. DE-A-36 28 508, published on 12th March 1987, describes human SOD in which the amino acids at positions 1, 6 and 111 may be modified. Corresponding patent applications have been published in Italy, Spain and Sweden.

Cysteine modifications have been made in other molecules. For instance, U.S. Pat No. 4,518,584 describes muteins of lymphokines in which cysteines that are not essential for biological activity are replaced with a neutral amino acid to facilitate the production of molecules with proper disulfide bridging in bacteria. Perry and Wetzel, Science (1984) 226:555-557 describe a mutein of T4 lysosyme having an Ile → Cys substitution at position three, a disulfide link between positions 3 and 97, and a free cysteine at position 54. Alkylation of that free cysteine increased the thermal stability of the mutein.

In sum the art suggests that alkylation of Cys 111 of native hSOD increases thermal stability, but is silent as to whether an amino acid substitution at position 111 or other positions would result in a functional molecule and whether such a change would increase the thermostability of the molecule. Indeed, the only reported mutein of hSOD lacked dismutase activity and the relative thermostabilities of yeast, bovine and human SODs appear to indicate that thermostability is dependent upon something other than the free cysteine content of the molecule.

### Disclosure of the Invention

Applicants have found that the substitution of either or both of the free cysteines at positions 6 and 111 of hSOD with an uncharged (neutral) amino acid enhances the thermal stability of the molecule. hSOD with both of these cysteines replaced is significantly more thermostable than hSOD with only a single cysteine replaced. Hence, hSOD with both cysteines replaced is preferred.

Accordingly, muteins of hSOD in which at least one of the cysteine residues at positions 6 and 111 is replaced with an uncharged amino acid are one aspect of the invention.

Another aspect of the invention is a method of enhancing the thermostability of hSOD comprising replacing at least one of the cysteine residues at positions 6 and 111 of hSOD with an uncharged amino acid.

Pharmaceutical and cosmetic compositions containing such muteins are yet another aspect of the invention.

The DNA, expression vectors and recombinant organisms that are used to make the above described muteins are also part of the invention.

### Brief Description of the Drawings

Figure 1 shows the sequence of a DNA fragment that encodes hSOD and the amino acid sequence of hSOD.

Figure 2 is a flow chart for the construction of plasmid pYSODA, a yeast expression plasmid used to produce a mutein of hSOD having a Cys → Ala substitution at position 6 of the amino acid sequence shown in Figure 1 (the mutein is designated hSOD Ala6).

Figure 3 is a flow chart for the construction of plasmid pSODCF1, an intermediate used in the construction of plasmid pYSODAS described below.

Figure 4 is a flow chart for the construction of the mutant M13 clone M13rp8SODC111S, an intermediate construct used to prepare plasmid pYSODS described below.

Figure 5 is a flow chart for the construction of plasmid pYSODS, a yeast expression plasmid used to produce a mutein of hSOD having a Cys → Ser substitution at amino acid position 111 of the sequence shown in Figure 1 (the mutein is designated hSOD Ser111).

Figure 6 is a flow chart for the construction of plasmid pYSODAS, a yeast expression plasmid for producing a mutein of hSOD having a Cys → Ala substitution at position 6 and a Cys → Ser substitution at position 111 (the mutein is designated hSOD Ala6 Ser111).

Figures 7-9 are photographs of various electrophoretic gel analyses described in the examples.

Figures 10-12 are graphs depicting the relative thermal stabilities of hSOD and the hSOD muteins described in the examples.

### Modes for Carrying Out the Invention

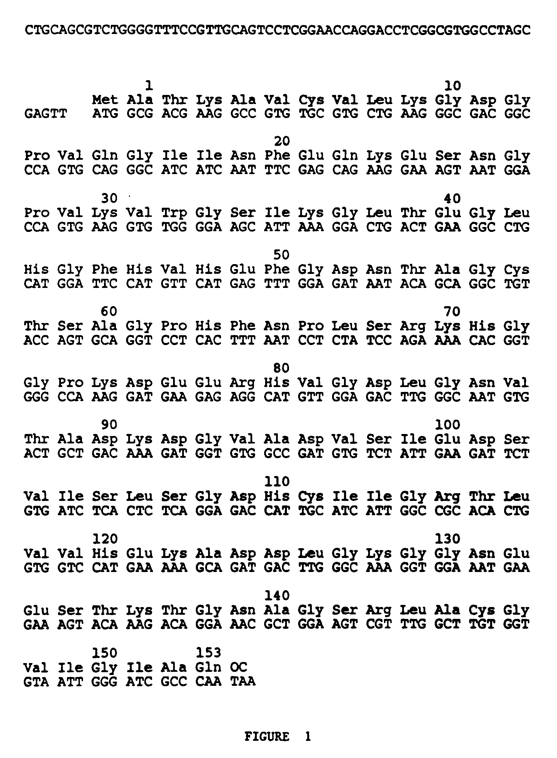

The hSOD muteins of the invention have at least one of the two free cysteine residues at positions 6 and 111 (amino acid numbering herein refers to that of the native molecule shown in Figure 1) replaced with an uncharged (nonpolar or uncharged polar), preferably acyclic (nonaromatic, nonheterocyclic) amino acid. Amino acids having hydrogen (i.e., glycine), an aliphatic, or a hydroxylic side chain are particularly preferred replacements. When both cysteines are replaced, the two replacement amino acids may be the same or different. Examples of amino acids that may be used to replace the cysteines are glycine, alanine, valine, leucine, isoleucine, serine, threonine, asparagine, glutamine, methionine, proline, tryptophan, and tyrosine. Serine and alanine are preferred replacement residues. Other amino acids of the native hSOD sequence may be replaced or deleted provided that such alterations neither affect adversely the dismutase activity of the mutein nor its thermostability. The number of such alterations will usually be less than about 5.

Specific examples of muteins of the invention are hSOD Ala6, hSOD Gly6, hSOD Val6, hSOD Leu6, hSOD Ile6, hSOD Thr6, hSOD Ser6, hSOD Gln6, hSOD Asn6, hSOD Met6, hSOD Ser111, hSOD Ala111, hSOD Gly111, hSOD Val111, hSOD Leu111, hSOD Ile111, hSOD Thr111, hSOD Gln111, hSOD Asn111, hSOD Met111, hSOD Gly6 Gly111, hSOD Ala6 Ala111, hSOD Leu6 Leu111, hSOD Ile6 Ile111, hSOD Ser6 Ser111, hSOD Thr6 Thr111, hSOD Gln6 Gln111, hSOD Asn6 Asn111, hSOD Met6 Met111, hSOD Gly6 Ala111, hSOD Gly6 Val111, hSOD Gly6 Leu111, hSOD Gly6 Ile111, hSOD Gly6 Ser111, hSOD Gly6 Thr111, hSOD Gly6 Gln111, hSOD Gly6 Asn111, hSOD Gly6 Met111, hSOD Ala6 Gly111, hSOD Ala6 Val111, hSOD Ala6 Leu111, hSOD Ala6 Ile111, hSOD Ala6 Ser111, hSOD Ala6 Thr111, hSOD Ala6 Gln111, hSOD Ala6 Asn111, hSOD Ala6 Met111, hSOD Val6 Gly111, hSOD Val6 Ala111, hSOD Val6 Leu111, hSOD Val6 Ile111, hSOD Val6 Ser111, hSOD Val6 Thr111, hSOD Val6 Gln111, hSOD Val6 Asn111, hSOD Val6 Met111, hSOD Leu6 Gly111, hSOD Leu6 Ala111, hSOD Leu6 Val111, hSOD Leu6 Ile111, hSOD Leu6 Ser111, hSOD Leu6 Thr111, hSOD Leu6 Gln111, hSOD Leu6 Asn111, hSOD Leu6 Met111, hSOD Ile6 Gly111, hSOD Ile6 Ala111, hSOD Ile6 Val111, hSOD Ile6 Leu111, hSOD Ile6 Ser111, hSOD Ile6 Thr111, hSOD Ile6 Gln111, hSOD Ile6 Met111, hSOD Ser6 Gly111, hSOD Ser6 Ala111, hSOD Ser6 Val111, hSOD Ser6 Leu111, hSOD Ser6 Ile111, hSOD Ser6 Thr111, hSOD Ser6 Gln111, hSOD Ser6 Asn111, hSOD Ser6 Met111, hSOD Thr6 Gly111, hSOD Thr6 Ala111, hSOD Thr6 Leu111, hSOD Thr6 Ile111, hSOD Thr6 Gln111, hSOD Thr6 Asn111, hSOD Thr6 Met111, hSOD Glu6 Gly111, hSOD Gln6 Ala111, hSOD Gln6 Val111, hSOD Glu6 Leu111, hSOD Gln6 Ser111, hSOD Glu6 Thr111, hSOD Glu6 Asn111, hSOD Glu6 Met111, hSOD Asn6 Gly111, hSOD Asn6 Ala111, hSOD Asn6 Val111, hSOD Asn6 Ser111, hSOD Asn6 Thr111, hSOD Asn6 Met111, hSOD Met6 Gly111, hSOD Met6 Ala111, hSOD Met6 Val111, hSOD Met6 Ser111, hSOD Met6 Thr111, hSOD Met6 Glu111, and hSOD Met6 Asn111.

The N-terminus of the mutein may be acetylated (as in native hSOD) or lack acetylation depending upon the organism in which the mutein is produced. Bacterially produced mutein will lack such acetylation whereas mutein produced in yeast using the procedures described in EPA 84111416.8 are so acetylated. Muteins having such acetylation are preferred. Similarly, the mutein may be glycosylated or unglycosylated depending upon the organism and signaling sequence with which it is produced.

Genes encoding the muteins of the invention may be made via oligonucleotide synthesis and ligation, site directed mutagenesis of the DNA sequence shown in Figure 1 and/or by insertion of synthetic DNA fragments that encode the desired amino acid substitutions into a DNA sequence encoding native hSOD. Site directed mutagenesis techniques are well known in the art. See for instance, Smith and Gilliam in Genetic Engineering Principles and Methods, Plenum Press (1981) 3:1-32; Zoller and Smith, Nucleic Acids Res (1982) 10:6487-6500; and Brake et al, Proc Natl Acad Sci USA (1984) 81:4642-4646. The mutant genes may be inserted into suitable prokaryotic or eukaryotic replicons (a genetic element such as a plasmid, a chromosome, or a virus that behaves as an autonomous unit of polynucleotide replication within a cell), the resulting expression vectors incorporated into suitable host organisms or cells, the recombinant organism or cell grown under conditions that result in expression of the mutant gene, and the resulting mutein isolated from the host or, if secreted, from the growth medium using the same techniques as are described in EPA 841111416.8 to produce recombinant hSOD. The disclosure of that EPA is incorporated herein by reference.

In creating an expression vector, the mutant sequence is located in the vector with the appropriate control DNA sequences, which include a promoter, a ribosomal binding site, and transcriptional and translational stop codons. The positioning and orientation of the coding sequence with respect to the control sequences is such that the coding sequence is transcribed under the "control" of the control sequences: i.e., the promoter will control the transcription of the mRNA derived from the coding sequence, the ribosomes will bind at the ribosomal binding site to begin the translational process, and the stop codon used to terminate translation will be upstream from the transcriptional termination codon. In addition to control sequence, it may be desirable to add regulatory sequences which allow for regulation of the expression of the mutant hSOD gene relative to the growth of the host cell.

The hSOD muteins of the invention may be used for the same purposes as hSOD. Because of their better thermostability, the muteins are more readily formulated with materials that require the use of elevated temperatures for blending or processing. The muteins may be used in human or veterinary medicine to treat (i.e. cure, alleviate or prevent) a variety of conditions. They are useful as antiinflammatory agents, chemopreventive agents to prevent oncogenesis and tumor promotion, protective agents to reduce cytotoxic and cardiotoxic effects of anticancer drugs or protect ischemic tissue. Like native hSOD, the muteins catalyze the reduction of superoxide radicals to hydroperoxide and molecular oxygen and may thus be used to reduce perfusion injury following ischemia, prolong the viability of excised isolated organ transplants, reduce injury on reperfusion following organ transplant or spinal cord ischemia, reduce cardiac infarct size, reduce spinal cord injury and treat bronchial pulmonary dysplasia.

For medical applications the mutein may be administered orally or parenterally to individuals in various dosage forms such as tablets, capsules, and injectables. When used to treat tissues in vitro the mutein will be added to the perfusion or culture medium. The mutein may be administered neat or admixed in effective amounts with pharmaceutically acceptable solid, semisolid or liquid vehicles such as albumins, globulins, dextran, Ficoll polymers, sugars, starches, and liposomes. Preferably the hSOD mutein is conveniently stored lyophilized with sugar, usually sucrose, usually in a ratio of 1:2 w/w. The lyophilized enzyme is conveniently reconstituted in a suitable diluent for the particular application. For example, to treat inflammatory joint disease the hSOD mutein may be reconstituted in physiologic saline in a volume convenient for intraarticular administration.

The dose of hSOD mutein administered to an individual will depend upon the nature of the individual being treated, the mode of treatment and the condition being treated. In general the amount administered must be sufficient to provide an enzymatically effective amount of the mutein at the desired site of treatment. In this regard when the mutein is administered systemically, larger doses will typically be required than when the mutein is administered locally at the site that requires treatment. By way of example, human patients having inflammatory joint disease are treated by a weekly intraarticular injection into a joint afflicted with the disease of a solution having hSOD mutein in a suitable diluent in an amount effective to reduce inflammation, usually 1 to 10 mg, more usually 2 to 6 mg. The injections are given weekly for a period of time sufficient to reduce inflammation, usually for 2 to 8 weeks, more usually for 4 to 6 weeks. Because the articular capsule limits leakage of the high molecular weight compound each afflicted joint should be treated with the required dosage. When used to minimize postischemic tissue damage the human patient is administered 10 mg to 1,000 mg, more usually 50 mg to 500 mg of hSOD mutein in a suitable diluent during the ischemic reaction. When the patient suffers ischemia due to a disease the solution is administered intraveneously or intraarterially as a bolus dosage or a continuous infusion. In such situations the hSOD mutein may be administered in conjunction with fibrinolytic agents such as urokinase, streptokinase or tissue plasminogen activator (TPA). When ischemic damage is due to a surgical procedure, hSOD mutein is administered during surgery. This application finds particular use in organ transplant surgery where hSOD is preferably administered prior to reirrigation of the organ and is also useful in any other surgery where bloodflow to an organ is interrupted, such as open heart surgery.

The hSOD muteins may also be used in cosmetic compositions for skin or hair care as a protective agent for keratinic substances or to prevent oxidative degradation of components of such compositions. For instance they may be added to such formulations to maintain or improve skin or hair qualities such as softness, flexibility or elasticity or prevent oxidation of oxidizable or self-oxidizable substances such as dyes used in cosmetic preparations. The form of the mutein-containing cosmetic formulation may be solid (e.g. cleansing bars), semisolid (e.g. creams, gels, ointments) or liquid (e.g. sprays, lotions, shampoos). The amount of hSOD mutein in such formulations will normally be in the range of about 0.01% to 5% by weight, more usually 0.05% to 1% by weight. In addition to the hSOD mutein, the cosmetic preparation may contain: fatty carrier materials such as natural oils (e.g. olive oil, avocado oil, and the like), fatty acid esters such as stearin, glyceryl monostearate, ethyl palmitate, cetyl myristate, isopropyl oleate and the like, alcohols such as cetyl alcohol or polyoxyethylenated fatty alcohols, waxes such as beeswax or synthetic waxes; dyes, perfumes, surfactants, preservatives, thickeners, or other additives conventionally included in cosmetics.

They may also be added to foods as preservatives to prevent oxidative degradation of food components.

The following examples further illustrate the invention. These examples are not intended to limit the invention in any manner.

### Construction of Yeast Plasmid pYSODA Encoding hSOD Ala6 Expression

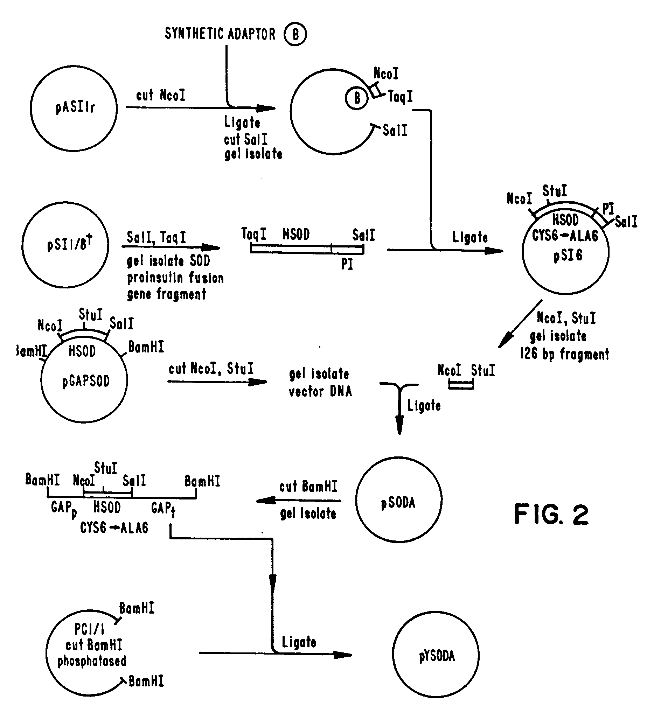

The construction of plasmid pYSODA is depicted in Figure 2.

A DNA adaptor fragment (designated B in Figure 2) having the sequence shown below was synthesized. 
NcoI 1 2 3 4 5 6 TaqI
MetAlaThrLysAlaValAlaValLeuLysGlyAspGlyProValGlnGlyIleIleAsnPh
CATGGCTACAAAGGCTGTTGCTGTTTTGAAGGGTGACGGCCCGTTCAAGGTATTATTAACTT
CGATGTTTCCGACAACGACAAAACTTCCCACTGCCGGGCAAGTTCCATAATAATTGAAGC
The fragment was phosphorylated on the coding (upper) strand only and was designed to encode the N-terminal sequence of hSOD with the codon for amino acid 6 altered to encode an alanine rather than a cysteine and have an NcoI site at its 5ʹ end and a TaqI site at its 3ʹ end.

Plasmid pASI1r was linearized with NcoI and ligated to adaptor B. Plasmid pASI1r consists of the expression cassette of plasmid pASI1 (the hSOD gene fused to the N-terminus of the human proinsulin gene with a methionine codon at the junction of the two genes under the regulation of a hybrid inducible S. cerevisae alcohol dehydrogenase 2-glyceraldehyde phosphate dehydrogenase (ADH2-GAP) promoter and the GAP terminator) in the S.R. 322 vector. Details of the composition and construction of plasmid pASI1 are described in commonly owned European Patent Application Serial No. 86104066.5, the disclosure of which as it relates to pASI1 is incorporated herein by reference. (The plasmid is designated pYASI1 in Serial No. 86104066.5.) The S.R. 322 vector is described in J Biol Chem (1985) 260:4384-4389. The ligation product was cut with SalI and the resulting large fragment was gel purified.

Plasmid pSI1/8 was cut with SalI and TaqI and a 660 bp TaqI - SalI fragment consisting of a portion of the hSOD gene fused to the N-terminus of the human proinsulin gene was gel isolated from the digest. Plasmid pSI1/8 contains the hSOD gene fused to the N-terminus of the human proinsulin gene under the regulation of the GAP promoter and terminator. Details of its composition and construction are described in commonly owned European Patent Application Serial No. 86104066.5, the disclosure of which, as it relates to pSI1/8, is incorporated herein by reference. (The plasmid is designated pYSI1 in Serial No. 86104066.5.)

The 660 bp TaqI - SalI fragment was ligated to the linear pASI1r - adaptor B construct to produce plasmid pSI6. That plasmid was cut with NcoI and StuI and a 126 bp fragment gel isolated from the digest.

The plasmid pGAPSOD was cut with NcoI and StuI and vector DNA was gel purified. pGAPSOD is a yeast promoter vector containing the hSOD gene under the regulation of the GAP promoter and terminator. Details of its composition and construction are described in commonly owned European Patent Application Serial No. 84111416.8, the disclosure of which, as it relates to this plasmid, is incorporated herein by reference. (The plasmid is designated pPGAPSOD in Serial No. 84111416.8.) The 126 bp NcoI - StuI fragment from pSI6 was ligated with the vector DNA to form plasmid pSODA. pSODA was then cut with BamHI and a BamHI expression cassette having the hSOD gene with an Ala codon at amino acid 6 flanked by GAP promoter and terminator sequences was isolated. This expression cassette was then cloned in the expression orientation in plasmid pCl/1, which had been digested with BamHI and phosphatased, to produce pYSODA. Plasmid pCl/1 is a derivative of pJDB219 (Beggs, Nature (1978) 275:104) in which the region corresponding to bacterial plasmid pMB9 in pJDB219 is replaced by pBR322.

### Construction of Yeast Plasmid pYSODS encoding hSOD Ser111 Expression

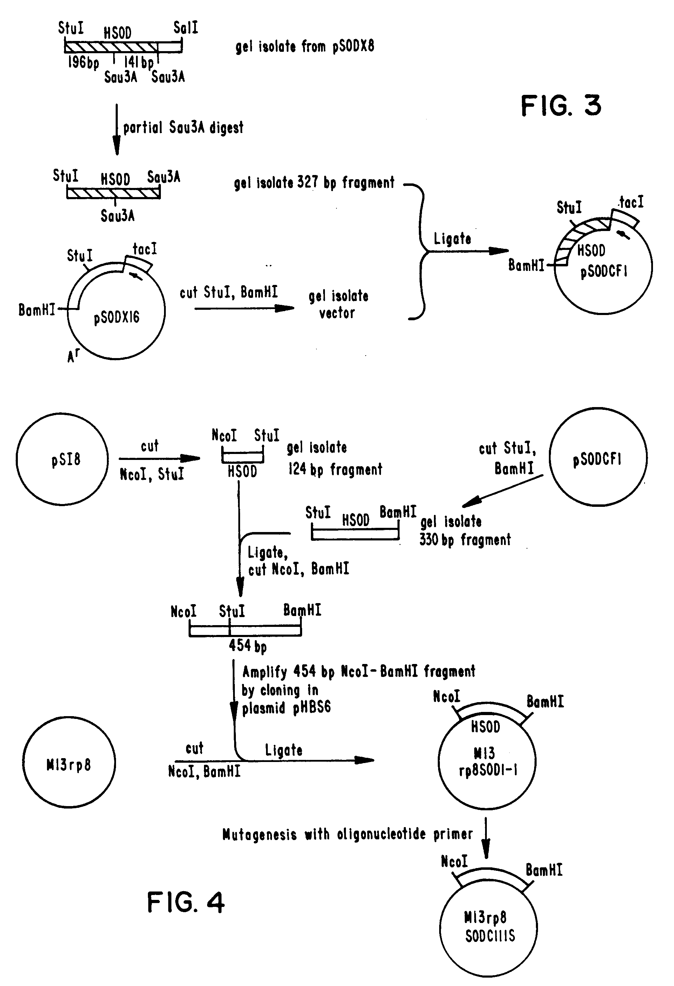

The mutagenesis of the hSOD Cys111 codon to a ser-encoding triplet was accomplished using M13 site directed mutagenesis. The construction of the mutant M13 plasmid and related plasmids involved in the construction of pYSODS are depicted in Figures 3 and 4 and described below.

Plasmid pSODX8 (described in Nucleic Acids Res (1985) 13:2017-2034) was cut with StuI and SalI. An approximately 400 bp fragment was gel isolated and partially digested with Sau3A and a 327 bp hSOD fragment was recovered. Plasmid pSODX16 (described in Nucl Acids Res (1985) 13:2017-2033) was cut with StuI and BamHI and the large vector fragment was gel isolated from the digest. This vector fragment was ligated to the 327 bp hSOD fragment to produce plasmid pSODCF1.

Plasmid pSI8, an hSOD-proinsulin fusion expression plasmid derived from pYASI1 (see above) and consisting of the expression cassette of pYASI1 in the S.R. 322 vector was cut with NcoI and StuI and a 124 bp fragment was gel isolated from the digest. That fragment was ligated with a 330 bp fragment isolated from pSODCF1 digested with StuI and BamHI to yield a 454 bp NcoI - BamHI fragment. That fragment was amplified by cloning in plasmid pHBS6 (described in European Patent Application Serial No. 85105405.6, the disclosure of which, as it relates to pHBS6, is incorporated herein by reference).

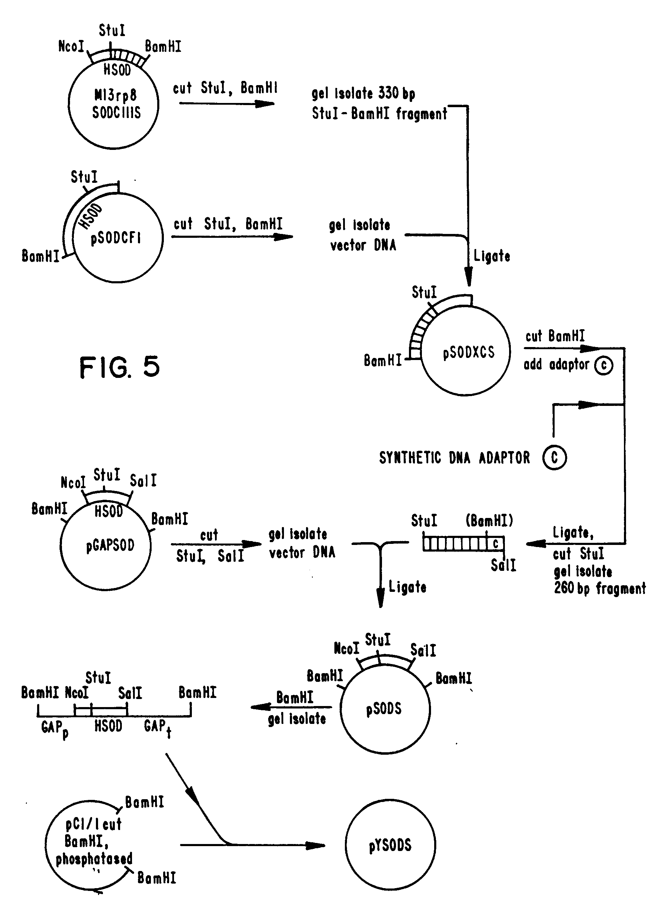

The phage vector M13rp8 was cut with NcoI and BamHI and the resulting vector fragment ligated to the 454 bp hSOD fragment to produce the vector M13rp8SOD-1. Single-stranded DNA was prepared from this vector to serve as a template for site directed in vitro mutagenesis using the following synthetic oligonucleotide primer 5'-GCCAATGATGGAATGGTCTCCTTGAGAGTGATATCACAGAATC-3'.
The mutagenesis was carried out according to the procedure of Brake et al, supra. The mutant RF M13 clone containing the hSOD Cys111 gene (designated M13rp8SODC111S) was identified by screening with a ³²P-labeled probe having the sequence 5'-TTGAGAGTGATATCACAGAATC-3'.
The mutant M13 clone M13rp8SODC111S was cut with StuI and BamHI and a 330 bp StuI - BamHI fragment isolated from the digest. That fragment was ligated to a vector fragment prepared by digesting pSODCF1 with StuI and BamHI. The resulting plasmid, pSODXCS, was digested with BamHI and a synthetic adaptor (designated (C) in Figure 5) having the sequence given below was ligated to the vector 
BamHI                                                                    SalI
 yIleAlaGlnOC
 GATCGCCCAATAAACATTCCCGGGATGTAGTCTGAGGCCCCTTAACTCATCTGTTATCCTGCTAG
     CGGGTTATTTGTAAGGGCCCCTACATCAGACTCCGGGGAATTGAGTAGACAATAGGACGATCAGCT
The ligation product was then cut with StuI and a 260 bp StuI - SalI fragment was gel isolated. This fragment was then ligated to vector DNA prepared by digesting pGAPSOD with StuI and SalI to produce the plasmid pSODS. pSODS was then cut with BamHI and a fragment consisting of the hSOD Cys111 gene flanked by GAP promoter and terminator sequences was isolated from the digest. That fragment was ligated into the vector pCl/1 that had been cut with BamHI and phosphatased to produce the plasmid pYSODS.

### Construction of Yeast Expression Plasmid pYSODAS for Expression of hSOD Ala6 Ser111

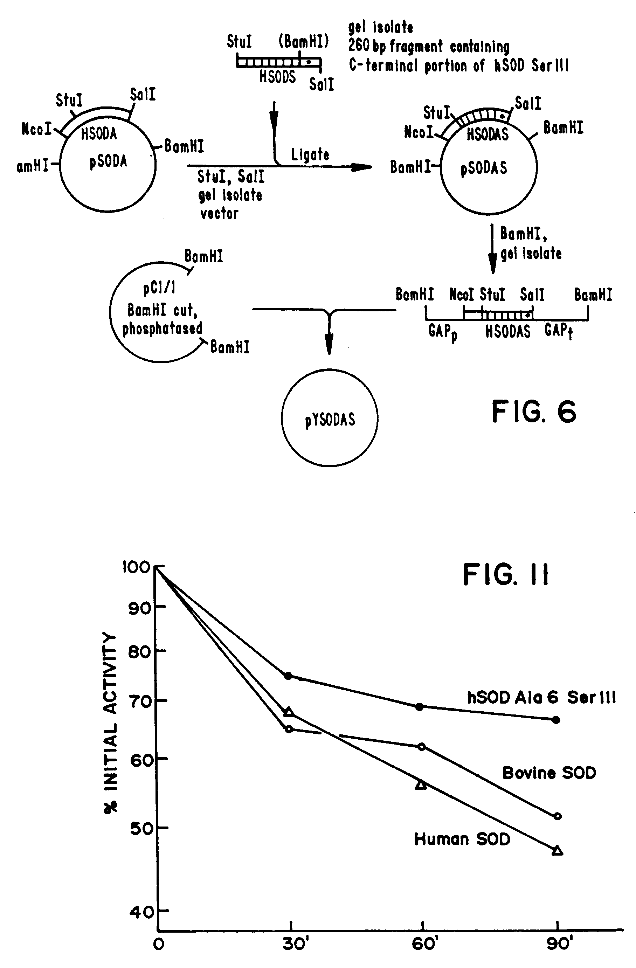

The construction of pYSODAS is diagrammed in Figure 6.

The above-described 260 bp fragment consisting of the BamHI - StuI fragment from pSODXCS with the adaptor (C) ligated to the BamHI end thereof was cloned into pSODA cut with StuI and SalI. The resulting plasmid pSODAS was cut with BamHI to produce a fragment consisting of the gene for hSOD Ala 6 Ser111 flanked by GAP promoter and terminator sequences. That fragment was inserted into BamHI cut pCl/1 to produce pYSODAS.

### Preparation of Recombinant Yeast Strains

Plasmids pYSODA, pYSODS, and pYSODAS were transformed into yeast strain 2150-2-3 as described in PNAS USA (1978) 75:1929-1933 to produce recombinant strains that produce hSOD Ala6, hSOD Ser111 and hSOD Ala6 Ser111.

### Yeast Growth and hSOD Mutein Purification

Ten liter cultures of yeast strains producing hSOD Ala6, hSOD Ser111 and hSOD Ala6 Ser111 were grown by inoculating 10 L of YEPD medium containing 3 mM CuSO₄ with 500 ml of starter culture in minimal glucose medium lacking leucine (Sherman, F., Fink, G.R., & Hicks, J.B., Methods in Yeast Genetics, (1982), Cold Spring Harbor, New York) supplemented with 50 µM CuSO₄ and grown to OD₆₅₀ ∼ 20.

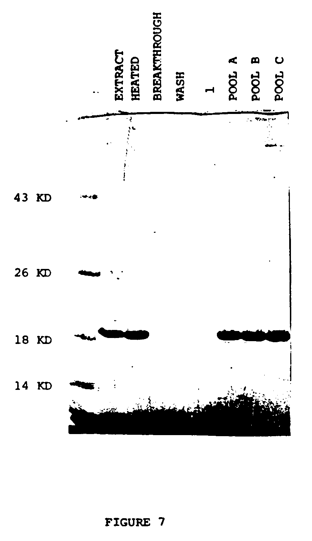

Yeast pastes (100-250 g wet weight) were lysed in 20 mM Tris-HCl, pH 8 (buffer), using a Dynomill Bead Disrupter. The lysate was centrifuged, and the pellet washed twice with buffer. The washes wre pooled with the original supernatant. After centrifugation, approximately 1 liter of extract was obtained, adjusted to pH 8 with NaOH, and stored at -20°C. The extract was thawed and heated for 2 hr at 65°C. After centrifugation to remove the precipitated protein and debris, 850 ml of clarified extract was obtained. Buffer was added to final volume of approximately 8.5 liters, resulting in a conductivity of 1.2 mmho. This was loaded onto a 400 ml column of DEAE-Sepharose. The column was washed with the same buffer, and eluted with a gradient up to 0.15 M NaCl in buffer. The peak was collected in 3 pools: A, which contains the bulk of the material from the center of the peak; B, containing the early side fractions; and C, containing the later side fractions. The pools were sterile filtered and aliquots stored at 4°C and at -20°C. Photographs of the gels of these fractions are shown in Figure 7.

The procedure resulted in a 2.5-fold purification, yielding protein at least 95% pure (see figure 7). Due to the high expression level (approximately 40% of the total protein), 2.5-fold purification is all that is possible. The overall yield is ∼ 60%; the heating step resulted in an 83% yield, and the column ∼ 70%. The heating step is valuable in that it purifies the hSOD mutein, and also clarifies the extract which otherwise is turbid with debris that is difficult to remove. The DEAE-Sepharose column removes the remaining contaminating proteins and other nonproteinaceous contaminants such as lipid, carbohydrate, and nucleic acid.

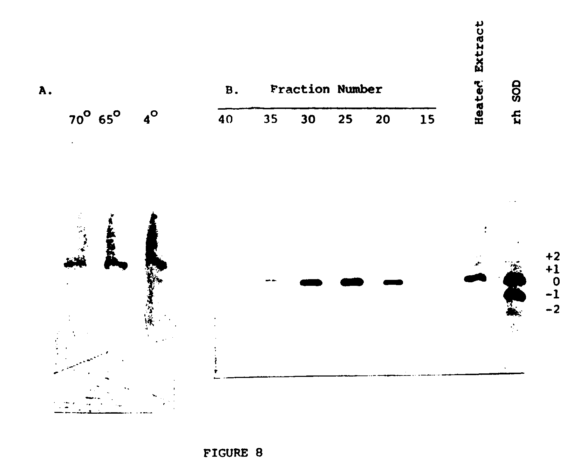

The purified hSOD mutein was compared to that present in the crude extract to ensure that not only was the activity present, but that the protein itself was unmodified. This is particularly important because of reactions that could occur during the heat step. By native gel electrophoresis in agarose gels, it is usually possible to separate 5 charged isomers to hSOD. These are called forms +2, +1, 0, -1 and -2. In fact, we found by native polyacrylamide gel and agarose gel electrophoresis, the hSOD Ala6 Ser111 is apparently unaltered after heating. As seen in Figure 8A, when extract is heated for 2 hr at 65 or 70°C, the pattern of bands seen after agarose gel electrophoresis is only slightly changed. This is a loss of some minor, very rapidly migrating species, but otherwise there is no change.

The specific activity of hSOD Ala6 Ser111 (3400 U/mg) is approximately the same as wild type hSOD (3500 U/mg) as assayed by the pyrogallol method, Involvement of the superoxide anion radical in the autooxidation of pyrogallol and a convenient assay for superoxide dismutase, Eur J Biochem (1974) 47:469-474).

### Thermal Stability of hSOD, hSOD Ala6, hSOD Ser111 and hSOD Ala6 Ser111

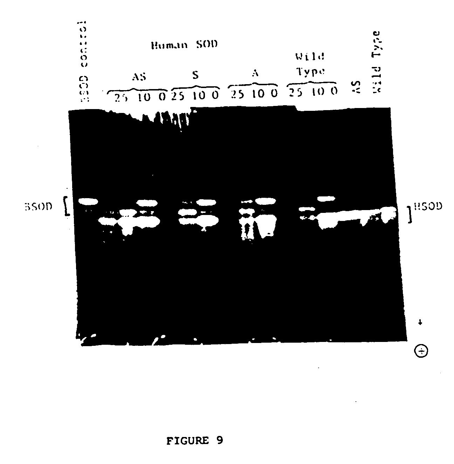

Analysis of the thermostability of the hSOD muteins made in yeast was performed by heating crude extracts at 80°C for 10 min and 25 min and comparing the superoxide dismutase activity of the heated SODs with that of the unheated control on an SOD activity gel as shown in Figure 9 (Beauchamp, C. and Fridovich, I., Analyt Biochem (1971) 44:276-287). The results depicted in Figure 9 indicate that hSOD Ala6 Ser111 is detectably more thermostable that hSOD and hSOD Ala6 and hSOD Ser111. hSOD Ala6 and hSOD Ser111 also appear to be more thermostable than wild type hSOD.

### Thermal Stability of hSOD Ala6 Ser111

### A. At pH 4.0.

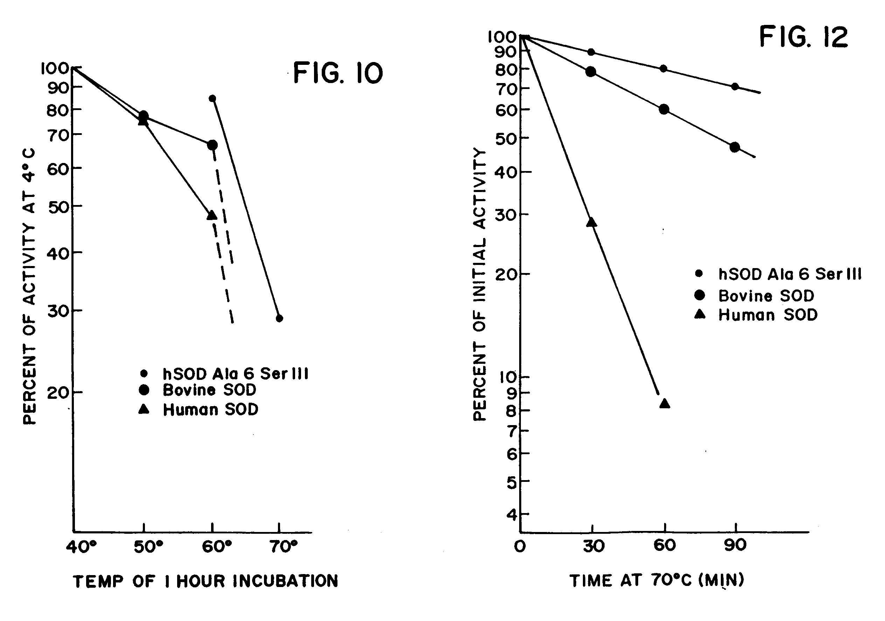

The thermal stabilities of purified recombinant hSOD, bovine SOD and hSOD Ala6 Ser111 were determined in 20 mM sodium acetate, pH 4, at a protein concentration of 20 µg/ml. After heating, the SOD activity was measured by the pyrogallol method. Figure 10 shows the melting curve seen after 1 hr of heating at temperatures from 50° to 70°C. Note that hSOD Ala6 Ser111 exhibits the most resistance to thermal denaturation under these conditions, followed by bovine SOD, and then by hSOD. After 1 hr at 60°C, hSOD Ala6 Ser111 has retained 86% of its activity, while the bovine SOD and hSOD have retained 68% and 48% activity, respectively.

Figure 11 shows the rate of denaturation at 60°C. Here the differences after 1 hr are less than the previous experiment (69% vs. 62% vs. 56%), but the relative stabilities are consistent with the earlier result.

The increased stability of the hSOD Ala6 Ser111 is especially evident after heating at 70°C (data not shown). Here, after 30 min, the hSOD Ala6 Ser111 retained 29% of its activity vs. ∼ 10% for the other SODs. And even after 90 min at 70°C, the hSOD Ala6 Ser111 still had 25% of its original activity while the others had less than 10% activity.

### B. At pH 7.8.

The experimental conditions were identical to those at pH 4, exzcept that the buffer was 0.1 M sodium phosphate, pH 7.8. Figure 12 shows the rates of inactivation at 70°C. Here, the differences in stability are dramatic; the hSOD Ala6 Ser111 is most stable, followed by bovine SOD and then hSOD. These are the same relative stabilities seen at pH 4, but at pH 7.8 the hSOD Ala6 Ser111 now has a 10-fold longer half-life at 70°C than hSOD (175 vs. 16 min). It is also more than 2-fold more stable than bovine SOD (175 vs. 84 min).

Modifications of the above described modes for carrying out the invention that are obvious to those of ordinary skill in the fields of protein chemistry, genetic engineering, medicine and related fields are intended to be within the scope of the following claims.

## Claims (Claims for the following Contracting State(s): AT, BE, CH, FR, GB, LI, LU, NL)

1. A mutein of human Cu/Zn superoxide dismutase in which at least one of the cysteine residues at positions 6 and 111 is replaced with another uncharged amino acid.

2. The mutein of claim 1 wherein said uncharged amino acid is acyclic.

3. The mutein of claim 2 wherein the side chain of the uncharged acyclic amino acid is hydrogen, an aliphatic side chain or a hydroxylic side chain.

4. The mutein of claim 1, 2, or 3 wherein the N-terminus of the mute in is acetylated.

5. The mutein of claim 1, 2, 3, or 4 wherein only one of said cysteine residues is replaced with another uncharged amino acid.

6. The mutein of claim 5 wherein the one of said cysteine residues is at position 6.

7. The mutein of claim 5 wherein the one of said cysteine residues is at position 111.

8. The mutein of claim 1, 4, 5, 6, or 7 wherein the uncharged amino acid is glycine, alanine, valine, leucine, isoleucine, serine, threonine, asparagine, glutamine, or methionine.

9. The mutein of claim 1, 2, 3, or 4 wherein both of the cysteine residues are replaced with another uncharged amino acid.

10. The mutein of claim 9 wherein the uncharged amino acid is glycine, valine, alanine, leucine, isoleucine, serine, threonine, asparagine, glutamine, or methionine.

11. The mutein of claim 9 wherein the cysteine residue at position 6 is replaced with alanine and the cysteine residue at position 111 is replaced with serine.

12. A method of enhancing the thermostability of human Cu/Zn superoxide dismutase comprising replacing at least one of the cysteine residues at positions 6 and 111 with another uncharged amino aid.

13. The method of claim 12 wherein only the cysteine residue at position 6 is replaced and the uncharged amino acid is serine.

14. The method of claim 12 wherein only the cysteine residue at position 111 is replaced and the uncharged amino acid is serine.

15. The method of claim 12 wherein the cysteine residue at position 6 is replaced with alanine and the cysteine residue at position 111 is replaced with serine.

16. A pharmaceutical composition comprising an enzymatically effective amount of the mutein of claim 1, 2, 3, 4, 5, 6, 7, 8, 9, 10, or 11 admixed with a pharmaceutically acceptable carrier.

17. A cosmetic composition comprising an enzymatically effective amount of the mutein of claim 1, 2, 3, 4, 5, 6, 7, 8, 9, 10, or 11 admixed with a carrier.

18. DNA encoding the mutein of claim 1, 2, 3, 4, 5, 6, 7, 8, 9, 10, or 11.

19. An expression vector for expressing the DNA of claim 18 in an organism comprising the DNA of claim 18 operably connected to DNA that enables the expression of the DNA of claim 18 in the organism.

20. A host organism containing the vector of claim 19 which permits the expression of said mutein-encoding DNA.

## Claims (Claims for the following Contracting State(s): DE, IT, SE)

1. A mutein of human Cu/Zn superoxide dismutase in which the cysteine residue at position 6 is replaced with alanine and the cysteine residue at position 111 is replaced with serine.

2. A method of enhancing the thermostability of human Cu/Zn superoxide dismutase comprising replacing the cysteine residue at position 6 with the alanine and replacing the cysteine residue at position 111 with serine.

3. A pharmaceutical composition comprising an enzymatically effective amount of the mutein of claim 1 admixed with a pharmaceutically acceptable carrier.

4. A cosmetic composition comprising an enzymatically effective amount of the mutein of claim 1, admixed with a carrier.

5. DNA encoding the mutein of claim 1.

6. An expression vector for expressing the DNA of claim 5 in an organism comprising the DNA of claim 5 operably connected to DNA that enables the expression of the DNA of claim 5 in the organism.

7. A host organism containing the vector of claim 6 which permits the expression of said mutein-encoding DNA.

## Claims (Claims for the following Contracting State(s): ES)

1. A method of enhancing the thermostability of human Cu/Zn superoxide dismutase comprising replacing the cysteine residue at positions 6 with alanine and the cysteine residue at position 111 with serine.

2. A method of preparing a pharmaceutical composition comprising admixing an enzymatically effective amount of the mutein as defined in claim 1 with a pharmaceutically acceptable carrier.

3. A method of preparing a cosmetic composition comprising admixing an enzymatically effective amount of the mutein as defined in claim 1 with a carrier.

4. A method of preparing human Cu/Zn superoxide dismutase in which the cysteine residue at position 6 is replaced with alanine and the cysteine residue at position 111 is replaced with serine which comprises providing a DNA which encodes said Cu/Zn superoxide dismutase in an expression vector wherein said DNA is operably linked to DNA that enables expression of said DNA in a host organism, transforming a host organism with said vector, and expressing said vector in said organism.

## Claims (Claims for the following Contracting State(s): GR)

1. A mutein of human Cu/Zn superoxide dismutase in which at least one of the cysteine residues at positions 6 and 111 is replaced with another uncharged amino acid.

2. The mutein of claim 1 wherein said uncharged amino acid is acyclic.

3. The mutein of claim 2 wherein the side chain of the uncharged acyclic amino acid is hydrogen, an aliphatic side chain or a hydroxylic side chain.

4. The mutein of claim 1, 2, or 3 wherein the N-terminus of the mute in is acetylated.

5. The mutein of claim 1, 2, 3, or 4 wherein only one of said cysteine residues is replaced with another uncharged amino acid.

6. The mutein of claim 5 wherein the one of said cysteine residues is at position 6.

7. The mutein of claim 5 wherein the one of said cysteine residues is at position 111.

8. The mutein of claim 1, 4, 5, 6, or 7 wherein the uncharged amino acid is glycine, alanine, valine, leucine, isoleucine, serine, threonine, asparagine, glutamine, or methionine.

9. The mutein of claim 1, 2, 3, or 4 wherein both of the cysteine residues are replaced with another uncharged amino acid.

10. The mutein of claim 9 wherein the uncharged amino acid is glycine, valine, alanine, leucine, isoleucine, serine, threonine, asparagine, glutamine, or methionine.

11. The mutein of claim 9 wherein the cysteine residue at position 6 is replaced with alanine and the cysteine residue at position 111 is replaced with serine.

12. A method of enhancing the thermostability of human Cu/Zn superoxide dismutase comprising replacing at least one of the cysteine residues at positions 6 and 111 with another uncharged amino aid.

13. The method of claim 12 wherein only the cysteine residue at position 6 is replaced and the uncharged amino acid is serine.

14. The method of claim 12 wherein only the cysteine residue at position 111 is replaced and the uncharged amino acid is serine.

15. The method of claim 12 wherein the cysteine residue at position 6 is replaced with alanine and the cysteine residue at position 111 is replaced with serine.

16. A cosmetic composition comprising an enzymatically effective amount of the mutein of claim 1, 2, 3, 4, 5, 6, 7, 8, 9, 10, or 11 admixed with a carrier.

17. DNA encoding the mutein of claim 1, 2, 3, 4, 5, 6, 7, 8, 9, 10, or 11.

18. An expression vector for expressing the DNA of claim 17 in an organism comprising the DNA of claim 17 operably connected to DNA that enables the expression of the DNA of claim 17 in the organism.

19. A host organism containing the vector of claim 18 which permits the expression of said muteinencoding DNA.

## Patentansprüche (Patentansprüche für folgende(n) Vertragsstaat(en): AT, BE, CH, FR, GB, LI, LU, NL)

1. Mutiertes Protein der menschlichen Cu/Zn Superoxid-Dismutase, in dem mindestens einer der Cysteinreste in Positionen 6 und 111 durch eine andere ungeladene Aminosäure ersetzt ist.

2. Mutiertes Protein nach Anspruch 1, in dem die ungeladene Aminosäure acyclisch ist.

3. Mutiertes Protein nach Anspruch 2, in dem die Seitenkette der ungeladenen, acyclischen Aminosäure ein Wasserstoffatom, eine aliphatische Seitenkette oder eine Hydroxylgruppen-haltige Seitenkette ist.

4. Mutiertes Protein nach Anspruch 1, 2 oder 3, in dem das N-terminale Ende des mutierten Proteins acetyliert ist.

5. Mutiertes Protein nach Anspruch 1, 2, 3 oder 4, in dem nur einer der Cysteinreste durch eine andere ungeladene Aminosäure ersetzt ist.

6. Mutiertes Protein nach Anspruch 5, in dem der eine Cysteinrest in Position 6 ist.

7. Mutiertes Protein nach Anspruch 5, in dem der eine Cysteinrest in Position 111 ist.

8. Mutiertes Protein nach Anspruch 1, 4, 5, 6 oder 7, in dem die ungeladene Aminosäure Glycin, Alanin, Valin, Leucin, Isoleucin, Serin, Threonin, Asparagin, Glutamin oder Methionin ist.

9. Mutiertes Protein nach Anspruch 1, 2, 3 oder 4, in dem beide Cysteinreste durch eine andere ungeladene Aminosäure ersetzt sind.

10. Mutiertes Protein nach Anspruch 9, in dem die ungeladene Aminosäure Glycin, Valin, Alanin, Leucin, Isoleucin, Serin, Threonin, Asparagin, Glutamin oder Methionin ist.

11. Mutiertes Protein nach Anspruch 9, in dem der Cysteinrest in Position 6 durch Alanin und der Cysteinrest in Position 111 durch Serin ersetzt sind.

12. Verfahren zur Erhöhung der Thermostabilität von menschlicher Cu/Zn Superoxid-Dismutase, umfassend das Ersetzen von mindestens einem der Cysteinreste in den Positionen 6 und 111 durch eine andere ungeladene Aminosäure.

13. Verfahren nach Anspruch 12, worin nur der Cysteinrest in Position 6 ersetzt wird und die ungeladene Aminosäure Serin ist.

14. Verfahren nach Anspruch 12, worin nur der Cysteinrest in Position 111 ersetzt wird und die ungeladene Aminosäure Serin ist.

15. Verfahren nach Anspruch 12, worin der Cysteinrest in Position 6 durch Alanin und der Cysteinrest in Position 111 durch Serin ersetzt werden.

16. Arzneimittel, umfassend eine enzymatisch wirksame Menge des mutierten Proteins nach Anspruch 1, 2, 3, 4, 5, 6, 7, 8, 9, 10 oder 11, vermischt mit einem pharmazeutisch verträglichen Träger.

17. Kosmetische Zusammensetzung, umfassend eine enzymatisch wirksame Menge des mutierten Proteins nach Anspruch 1, 2, 3, 4, 5, 6, 7, 8, 9, 10 oder 11, vermischt mit einem Träger.

18. DNA, codierend das mutierte Protein nach Anspruch 1, 2, 3, 4, 5, 6, 7, 8, 9, 10 oder 11.

19. Expressionsvektor zur Expression der DNA nach Anspruch 18 in einem Organismus, umfassend die DNA nach Anspruch 18, die funktionell mit DNA, die die Expression der DNA nach Anspruch 18 in dem Organismus ermöglicht, verknüpft ist.

20. Wirtsorganismus, enthaltend den Vektor nach Anspruch 19, der die Expression der DNA, die das mutierte Protein codiert, erlaubt.

## Patentansprüche (Patentansprüche für folgende(n) Vertragsstaat(en): DE, IT, SE)

1. Mutiertes Protein der menschlichen Cu/Zn Superoxid-Dismutase, in dem der Cysteinrest in Position 6 durch Alanin und der Cysteinrest in Position 111 durch Serin ersetzt sind.

2. Verfahren zur Erhöhung der Thermostabilität von menschlicher Cu/Zn Superoxid-Dismutase, umfassend das Ersetzen des Cysteinrests in Position 6 durch Alanin und des Cysteinrests in Position 111 durch Serin.

3. Arzneimittel, umfasssend eine enzymatisch wirksame Menge des mutierten Proteins nach Anspruch 1, vermischt mit einem pharmazeutisch verträglichen Träger.

4. Kosmetische Zusammensetzung, umfassend eine enzymatisch wirksame Menge des mutierten Proteins nach Anspruch 1, vermischt mit einem Träger.

5. DNA, codierend das mutierte Protein nach Anspruch 1.

6. Expressionsvektor zur Expression der DNA nach Anspruch 5 in einem Organismus, umfassend die DNA nach Anspruch 5, die funktionell mit einer DNA, die die Expression der DNA nach Anspruch 5 in dem Organismus ermöglicht, verknüpft ist.

7. Wirtsorganismus, enthaltend den Vektor nach Anspruch 6, der die Expression der DNA, die das mutierte Protein codiert, erlaubt.

## Patentansprüche (Patentansprüche für folgende(n) Vertragsstaat(en): ES)

1. Verfahren zur Erhöhung der Thermostabilität von menschlicher Cu/Zn Superoxid-Dismutase, umfassend das Ersetzen des Cysteinrests in Position 6 durch Alanin und des Cysteinrests in Position 111 durch Serin.

2. Verfahren zur Herstellung eines Arzneimittels, umfassend das Mischen einer enzymatisch wirksamen Menge des mutierten Proteins nach Anspruch 1 mit einem pharmazeutisch verträglichen Träger.

3. Verfahren zur Herstellung einer kosmetischen Zusammensetzung, umfassend das Mischen einer enzymatisch wirksamen Menge des mutierten Proteins nach Anspruch 1 mit einem Träger.

4. Verfahren zur Herstellung menschlicher Cu/Zn Superoxid-Dismutase, worin der Cysteinrest in Position 6 durch Alanin und der Cysteinrest in Position 111 durch Serin ersetzt werden, das die Bereitstellung einer DNA, die die Cu/Zn Superoxid-Dismutase in einem Expressionsvektor, in dem die DNA funktionell mit der DNA, die die Expression der DNA in einem Wirtsorganismus ermöglicht, verknüpft ist, die Transformation des Wirtsorganismus mit dem Vektor und die Expression des Vektors in dem Organismus umfaßt.

## Patentansprüche (Patentansprüche für folgende(n) Vertragsstaat(en): GR)

1. Mutiertes Protein der menschlichen Cu/Zn Superoxid-Dismutase, in dem mindestens einer der Cysteinreste in Positionen 6 und 111 durch eine andere ungeladene Aminosäure ersetzt ist.

2. Mutiertes Protein nach Anspruch 1, in dem die ungeladene Aminosäure acyclisch ist.

3. Mutiertes Protein nach Anspruch 2, in dem die Seitenkette der ungeladenen, acyclischen Aminosäure ein Wasserstoffatom, eine aliphatische Seitenkette oder eine Hydroxylgruppen-haltige Seitenkette ist.

4. Mutiertes Protein nach Anspruch 1, 2 oder 3, in dem das N-terminale Ende des mutierten Proteins acetyliert ist.

5. Mutiertes Protein nach Anspruch 1, 2, 3 oder 4, in dem nur einer der Cysteinreste durch eine andere ungeladene Aminosäure ersetzt ist.

6. Mutiertes Protein nach Anspruch 5, in dem der eine Cysteinrest in Position 6 ist.

7. Mutiertes Protein nach Anspruch 5, in dem der eine Cysteinrest in Position 111 ist.

8. Mutiertes Protein nach Anspruch 1, 4, 5, 6 oder 7, in dem die ungeladene Aminosäure Glycin, Alanin, Valin, Leucin, Isoleucin, Serin, Threonin, Asparagin, Glutamin oder Methionin ist.

9. Mutiertes Protein nach Anspruch 1, 2, 3 oder 4, in dem beide Cysteinreste durch eine andere ungeladene Aminosäure ersetzt sind.

10. Mutiertes Protein nach Anspruch 9, in dem die ungeladene Aminosäure Glycin, Valin, Alanin, Leucin, Isoleucin, Serin, Threonin, Asparagin, Glutamin oder Methionin ist.

11. Mutiertes Protein nach Anspruch 9, in dem der Cysteinrest in Position 6 durch Alanin und der Cysteinrest in Position 111 durch Serin ersetzt sind.

12. Verfahren zur Erhöhung der Thermostabilität von menschlicher Cu/Zn Superoxid-Dismutase, umfassend das Ersetzen von mindestens einem der Cysteinreste in den Positionen 6 und 111 durch eine andere ungeladene Aminosäure.

13. Verfahren nach Anspruch 12, worin nur der Cysteinrest in Position 6 ersetzt wird und die ungeladene Aminosäure Serin ist.

14. Verfahren nach Anspruch 12, worin nur der Cysteinrest in Position 111 ersetzt wird und die ungeladene Aminosäure Serin ist.

15. Verfahren nach Anspruch 12, worin der Cysteinrest in Position 6 durch Alanin und der Cysteinrest in Position 111 durch Serin ersetzt werden.

16. Kosmetische Zusammensetzung, umfassend eine enzymatisch wirksame Menge des mutierten Proteins nach Anspruch 1, 2, 3, 4, 5, 6, 7, 8, 9, 10 oder 11, vermischt mit einem Träger.

17. DNA, codierend das mutierte Protein nach Anspruch 1, 2, 3, 4, 5, 6, 7, 8, 9, 10 oder 11.

18. Expressionsvektor zur Expression der DNA nach Anspruch 17 in einem Organismus, umfassend die DNA nach Anspruch 17, die funktionell mit DNA, die die Expression der DNA nach Anspruch 17 in dem Organismus ermöglicht, verknüpft ist.

19. Wirtsorganismus, enthaltend den Vektor nach Anspruch 18, der die Expression der DNA, die das mutierte Protein codiert, erlaubt.

## Revendications (Revendications pour l'(les) Etat(s) contractant(s) suivant(s): AT, BE, CH, FR, GB, LI, LU, NL)

1. Mutéine de superoxyde dismutase Cu/Zn humaine, dans laquelle au moins l'un des résidus cystéine, se trouvant aux positions 6 et 111, est remplacé par un autre aminoacide non chargé.

2. Mutéine selon la revendication 1, dans laquelle ledit aminoacide non chargé est acyclique.

3. Mutéine selon la revendication 2, dans laquelle la chaîne latérale de l'aminoacide acyclique non chargé est un atome d'hydrogène, une chaîne latérale aliphatique ou une chaîne latérale hydroxylique.

4. Mutéine selon les revendications 1, 2 ou 3, dans laquelle l'extrémité amino-terminale de la mutéine est acétylée.

5. Mutéine selon les revendications 1, 2, 3, ou 4, dans laquelle seul l'un desdits résidus de cystéine est remplacé par un autre aminoacide non chargé.

6. Mutéine selon la revendication 5, dans laquelle ledit seul résidu de cystéine est en position 6.

7. Mutéine selon la revendication 5, dans laquelle ledit seul résidu de cystéine est en position 111.

8. Mutéine selon la revendication 1, 4, 5, 6 ou 7, dans laquelle l'aminoacide non chargé est la glycine, l'alanine, la valine, la leucine, l'isoleucine, la sérine, la thréonine, l'asparagine, la glutamine ou la méthionine.

9. Mutéine selon la revendication 1, 2, 3 ou 4, dans laquelle les deux résidus de cystéine sont remplacés par un autre aminoacide non chargé.

10. Mutéine selon la revendication 9, dans laquelle l'aninoacide non chargé est la glycine, la valine, l'alanine, la leucine, l'isoleucine, la sérine, la thréonine, l'asparagine, la glutamine ou la méthionine.

11. Mutéine selon la revendication 9, dans laquelle le résidu de cystéine en position 6 est remplacé par l'alanine et le résidu de cystéine en position 111 est remplacé par la sérine.

12. Procédé pour renforcer la thermostabilité de la superoxyde dismutase Cu/Zn humaine, comprenant les étapes consistant à remplacer au moins l'un des résidus de cystéine en positions 6 et 111 par un autre aminoacide non chargé.

13. Procédé selon la revendication 12, dans lequel seul le résidu de cystéine en position 6 est remplacé et l'aminoacide non chargé est la sérine.

14. Procédé selon la revendication 12, dans lequel seul le résidu de cystéine en position 111 est remplacé et l'aminoacide non chargé est la sérine.

15. Procédé selon la revendication 12, dans lequel le résidu de cystéine en position 6 est remplacé par l'alanine et le résidu de cystéine en position 111 est remplacé par la sérine.

16. Composition pharmaceutique comprenant une quantité enzymatiquement efficace de la mutéine selon l'une quelconque des revendications 1, 2, 3, 4, 5, 6, 7, 8, 9, 10 ou 11, en mélange avec un véhicule pharmaceutiquement acceptable.

17. Composition cosmétique comprenant une quantité enzymatiquement efficace de la mutéine selon l'une quelconque des revendications 1, 2, 3, 4, 5, 6, 7, 8, 9, 10 ou 11, en mélange avec un véhicule.

18. ADN codant pour la mutéine selon l'une quelconque des revendications 1, 2, 3, 4, 5, 6, 7, 8, 9, 10 ou 11.

19. Vecteur d'expression pour exprimer l'ADN selon la revendication 18 dans un organisme, comprenant l'ADN selon la revendication 18 lié de façon opérationnelle à l'ADN qui permet l'expression de l'ADN selon la revendication 18 dans l'organisme.

20. Organisme hôte contenant un vecteur selon la revendication 19, qui permet l'expression dudit ADN codant pour la mutéine.

## Revendications (Revendications pour l'(les) Etat(s) contractant(s) suivant(s): DE, IT, SE)

1. Mutéine de superoxyde dismutase Cu/Zn humaine, dans laquelle le résidu de cystéine en position 6 est remplacé par l'alanine et le résidu de cystéine en position 111 est remplacé par la sérine.

2. Procédé pour renforcer la thermostabilité de la superoxyde dismutase Cu/Zn humaine, consistant à remplacer le résidu de cystéine en position 6 par l'alanine et le résidu de cystéine en position 111 par la sérine.

3. Composition pharmaceutique comprenant une quantité enzymatiquement efficace de la mutéine selon la revendication 1, en mélange avec un véhicule pharmaceutiquement acceptable.

4. Composition cosmétique comprenant une quantité enzymatiquement efficace de la mutéine selon la revendication 1, en mélange avec un véhicule.

5. ADN codant pour la mutéine selon la revendication 1.

6. Vecteur d'expression pour exprimer l'ADN selon la revendication 5 dans un organisme, comprenant l'ADN selon la revendication 5 lié de façon opérationnelle à l'ADN qui permet l'expression de l'ADN selon la revendication 5 dans l'organisme.

7. Organisme hôte contenant le vecteur selon la revendication 6 qui permet l'expression dudit ADN codant pour la mutéine.

## Revendications (Revendications pour l'(les) Etat(s) contractant(s) suivant(s): ES)

1. Procédé pour renforcer la thermostabilité de la superoxyde dismutase Cu/Zn humaine, consistant à remplacer le résidu de cystéine en position 6 par l'alanine et le résidu de cystéine en position 111 par la sérine.

2. Procédé de préparation d'une composition pharmaceutique, consistant à mélanger une quantité enzymatiquement efficace de la mutéine selon la revendication 1 avec un véhicule pharmaceutiquement acceptable.

3. Procédé de préparation d'une composition cosmétique, consistant à mélanger une quantité enzymatiquement efficace de la mutéine selon la revendication 1 avec un véhicule.

4. Procédé de préparation de la superoxyde dismutase Cu/Zn humaine, dans laquelle le résidu de cystéine en position 6 est remplacé par l'alanine et le résidu de cystéine en position 111 est remplacé par la sérine, comprenant les étapes consistant à produire un ADN qui code pour ladite superoxyde dismutase Cu/Zn humaine, dans un vecteur d'expression dans lequel l'ADN est lié de façon opérationnelle à l'ADN qui permet l'expression dudit ADN dans un organisme hôte, à transformer l'organisme hôte à l'aide dudit vecteur, et à exprimer ledit vecteur dans ledit organisme.

## Revendications (Revendications pour l'(les) Etat(s) contractant(s) suivant(s): GR)

1. Mutéine de superoxyde dismutase Cu/Zn humaine, dans laquelle au moins l'un des résidus cystéine, se trouvant aux positions 6 et 111, est remplacé par un autre aminoacide non chargé.

2. Mutéine selon la revendication 1, dans laquelle ledit aminoacide non chargé est acyclique.

3. Mutéine selon la revendication 2, dans laquelle la chaîne latérale de l'aminoacide acyclique non chargé est un atome d'hydrogène, une chaîne latérale aliphatique ou une chaîne latérale hydroxylique.

4. Mutéine selon les revendications 1, 2 ou 3, dans laquelle l'extrémité amino-terminale de la mutéine est acétylée.

5. Mutéine selon les revendications 1, 2, 3, ou 4, dans laquelle seul l'un desdits résidus de cystéine est remplacé par un autre aminoacide non chargé.

6. Mutéine selon la revendication 5, dans laquelle ledit seul résidu de cystéine est en position 6.

7. Mutéine selon la revendication 5, dans laquelle ledit seul résidu de cystéine est en position 111.

8. Mutéine selon la revendication 1, 4, 5, 6 ou 7, dans laquelle l'aminoacide non chargé est la glycine, l'alanine, la valine, la leucine, l'isoleucine, la sérine, la thréonine, l'asparagine, la glutamine ou la méthionine.

9. Mutéine selon la revendication 1, 2, 3 ou 4, dans laquelle les deux résidus de cystéine sont remplacés par un autre aminoacide non chargé.

10. Mutéine selon la revendication 9, dans laquelle l'aninoacide non chargé est la glycine, la valine, l'alanine, la leucine, l'isoleucine, la sérine, la thréonine, l'asparagine, la glutamine ou la méthionine.

11. Mutéine selon la revendication 9, dans laquelle le résidu de cystéine en position 6 est remplacé par l'alanine et le résidu de cystéine en position 111 est remplacé par la sérine.

12. Procédé de renforcement de la thermostabilité de la superoxyde dismutase Cu/Zn humaine, comprenant les étapes consistant à remplacer au moins l'un des résidus de cystéine en positions 6 et 111 par un autre aminoacide non chargé.

13. Procédé selon la revendication 12, dans lequel seul le résidu de cystéine en position 6 est remplacé et l'aminoacide non chargé est la sérine.

14. Procédé selon la revendication 12, dans lequel seul le résidu de cystéine en position 111 est remplacé et l'aminoacide non chargé est la sérine.

15. Procédé selon la revendication 12, dans lequel le résidu de cystéine en position 6 est remplacé par l'alanine et le résidu de cystéine en position 111 est remplacé par la sérine.

16. Composition cosmétique comprenant une quantité enzymatiquement efficace de la mutéine selon l'une quelconque des revendications 1, 2, 3, 4, 5, 6, 7, 8, 9, 10 ou 11, en mélange avec un véhicule.

17. ADN codant pour la mutéine selon l'une quelconque des revendications 1, 2, 3, 4, 5, 6, 7, 8, 9, 10 ou 11.

18. Vecteur d'expression pour exprimer l'ADN selon la revendication 17 dans un organisme, comprenant l'ADN selon la revendication 17 lié de façon opérationnelle à l'ADN qui permet l'expression de l'ADN selon la revendication 17 dans l'organisme.

19. Organisme hôte contenant un vecteur selon la revendication 18, qui permet l'expression dudit ADN codant pour la mutéine.
